(19) Europäisches Patentamt
European Patent Office
Office européen des brevets

(11) EP 0 724 877 B1

(12) **FASCICULE DE BREVET EUROPEEN**

(45) Date de publication et mention de la délivrance du brevet:
**28.06.2000 Bulletin 2000/26**

(51) Int Cl.[7]: **A61K 31/215**, A61K 31/355

(21) Numéro de dépôt: **96400133.3**

(22) Date de dépôt: **19.01.1996**

(54) **Association de fénofibrate et de vitamine E, utilisation en thérapeutique**

Fenofibrat-Vitamin-E-Mischung und ihre therapeutische Verwendung

Mixture of fenofibrate and vitamin E and its therapeutic use

(84) Etats contractants désignés:
**AT BE CH DE DK ES FR GB GR IE IT LI LU MC NL PT SE**

(30) Priorité: **02.02.1995 FR 9501216**

(43) Date de publication de la demande:
**07.08.1996 Bulletin 1996/32**

(73) Titulaire: **LABORATOIRES FOURNIER S.A.**
**21000 Dijon (FR)**

(72) Inventeurs:
- **Edgar, Alan Dunlap**
  **F-21490 Saint-Julien (FR)**
- **Bellamy, François**
  **F-21910 Saulon-la-Rue (FR)**

(74) Mandataire: **Clisci, Serge et al**
**S.A. FEDIT-LORIOT & AUTRES**
**CONSEILS EN PROPRIETE INDUSTRIELLE**
**38, Avenue Hoche**
**75008 Paris (FR)**

(56) Documents cités:
**EP-A- 0 295 637**
**EP-A- 0 330 532**
**EP-A- 0 344 820**
**WO-A-94/15592**
**FR-A- 2 163 736**

- **PATENT ABSTRACTS OF JAPAN vol. 17, no. 627 (C-1131), 19 Novembre 1993 & JP-A-05 194209 (GRELAN PHARMACEUT CO LTD), 3 Août 1993,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 724 877 B1

## Description

### *Domaine de l'invention*

La présente invention a trait à une nouvelle association de fénofibrate et de vitamine E, cette nouvelle association étant utile en tant que médication anti-athérome et présentant une synergie en ce qui concerne la protection des lipoprotéines de faible densité (LDL) du plasma vis-à-vis de l'oxydation.

Elle a trait également à l'utilisation de cette nouvelle association synergétique en thérapeutique.

### *Art antérieur*

On sait que la vitamine E, qui répond à la nomenclature d'a-tocophérol ou de 3,4-dihydro-2,5,7,8-tétraméthyl-2-(4,8,12-triméthyltridécyl)-2H-1-benzopyran-6-ol et a pour formule développée :

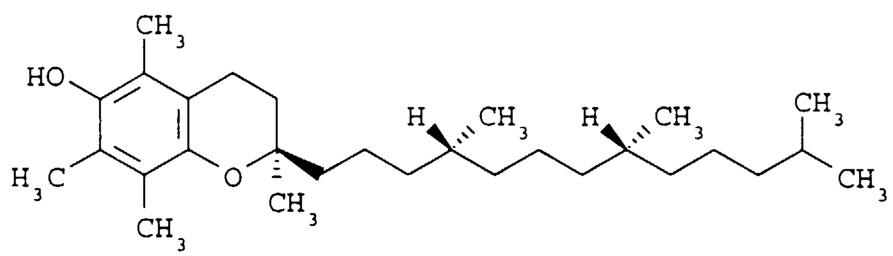

,

est une vitamine qui a des propriétés anti-oxydantes et qui est utilisée principalement soit telle quelle sous la forme d1 (i.e. forme dite naturelle) ou la forme d (i.e. le diastéréoisomère le plus actif), soit estérifiée notamment par l'acide acétique.

L'acétate de vitamine E, qui est également dénommé acétate d'α-tocophérol ou acétate d'α-tocophéryle et a pour formule développée :

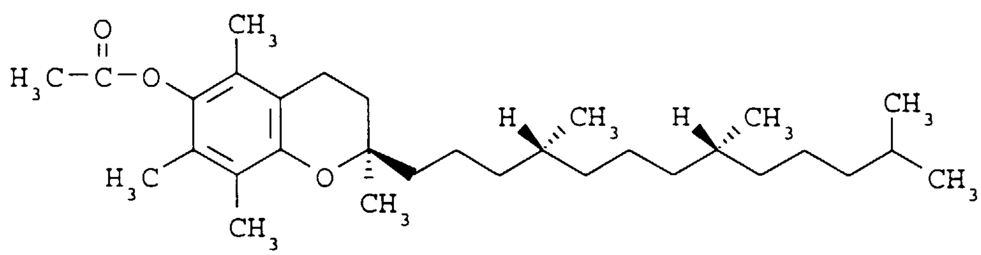

,

est principalement utilisé sous la forme d1 (i.e. le produit "standard") ou la forme d.

Selon le *Merck Index,* (1989), 11ème édition, pages 1579-1580, entrées No. 9931 "Vitamin E" et No. 9932 "Vitamin E Acetate", l'unité internationale (IU) de vitamine E est définie par rapport à l'acétate de d1-α-tocophérol. Ainsi on a les relations :

(1) 1mg d'acétate de d1-α-tocophérol = 1 IU
(2) 1mg d'acétate de d-α-tocophérol = 1,36 IU

On sait par ailleurs que la vitamine E et son acétate protègent, en tant qu'agents anti-oxydants liposolubles, les lipoprotéines plasmatiques et en particulier les lipoprotéines de faible densité (LDL) vis-à-vis de l'oxydation par les radicaux libres.

On sait que le fénofibrate, qui répond à la nomenclature systématique de 2-[4-(4-chlorobenzoyl)phénoxy]-2-méthylpropionate d'isopropyle ou (selon les *Chemical Abstracts*) d'acide2-[4-(4-chlorobenzoyl)phénoxy]-2-méthylpropionique 1-méthyléthyl ester et a pour formule développée :

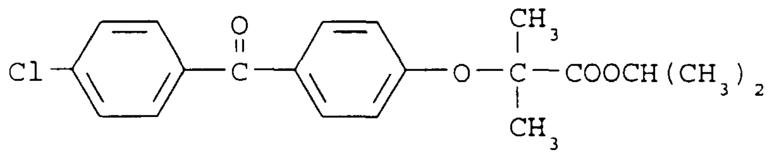

,

est un agent hypolipidémiant de référence possédant en outre la propriété d'abaisser la cholestérolémie et la triglycéridémie.

On connaît de EP-A-0 330 532 une solution technique permettant d'améliorer la biodisponibilité du fénofibrate, selon laquelle une dose unitaire de 200 mg de fénofibrate préalablement co-micronisé avec un agent tensio-actif solide est thérapeutiquement équivalente à une dose de 300 mg de fénofibrate micronisé en l'absence d'agent tensio-actif solide.

On sait enfin que la capacité du fénofibrate à protéger les lipoprotéines vis-à-vis de l'oxydation a été étudiée dans l'article intitulé "Antioxidant Therapy and Uptake of Human oxidized LDL by Macrophages" de J.C. FRUCHART et al., *Annals of the New York Academy of Sciences,* 1989 ; 570 : 447-448. Selon cet article, les LDL de patients hypercholestérolémiques ayant reçu pendant deux mois 300 mg/j de fénofibrate, 1000 mg/j de vitamine E (i.e. d1-α-tocophérol) ou le traitement combiné de 300 mg/j de fénofibrate et de 1000 mg/j de vitamine E ont été isolées puis oxydées par incubation (24h) avec du cuivre avant d'être transférées et incubées (5h) dans une culture de macrophages de péritoine de souris pour étude de la captation (en anglais : "uptake") des LDL.

Les résultats fournis dans ledit article indiquent que (i) la captation des LDL du lot de patients ayant reçu le fénofibrate est identique à celle du lot contrôle, ce qui signifie que ledit fénofibrate n'a aucun effet sur l'oxydation des LDL, et (ii) le taux de captation des LDL diminue de 19,9 % pour le groupe ayant reçu la vitamine E et de 22,4 % pour le lot ayant reçu l'association fénofibrate/vitamine E.

Il se trouve que, selon l'expérience de la Demanderesse, la durée d'incubation de 24h pour oxyder les LDL par le cuivre (selon J.C. FRUCHART et al.) est trop longue et que par suite les résultats obtenus sont peu précis et sujets à caution.

***But de l'invention***

Il existe un besoin en ce qui concerne une nouvelle médication pour le traitement et/ou la prévention des maladies athéromateuses.

Pour satisfaire ce besoin, on se propose de fournir une association de fénofibrate et de vitamine E qui soit vraiment efficace dans la protection des lipoprotéines plasmatiques, notamment, surtout les LDL et, le cas échéant, les lipoprotéines de très faible densité (VLDL), vis-à-vis de l'oxydation.

La solution technique que l'on préconise à cet effet fait appel à une association de fénofibrate et de vitamine E, dans laquelle

- le fénofibrate a été préalablement co-micronisé avec un agent tensio-actif solide, et
- le rapport Ra de la quantité de fénofibrate (exprimée en mg) à la quantité de vitamine E (exprimée en IU) est, à la différence de l'enseignement de l'article de J.C. FRUCHART et al. précité, supérieur à 0,3 mg/IU.

***Objet de l'invention***

La nouvelle solution technique repose sur deux découvertes. La première est que si le fénofibrate non co-micronisé n'a pratiquement aucun effet protecteur sur les lipoprotéines plasmatiques LDL vis-à-vis de l'oxydation selon J.C. FRUCHART et al., en revanche le fénofibrate co-micronisé avec un agent tensio-actif solide protège lesdites lipoprotéines vis-à-vis de l'oxydation.

La seconde découverte est que l'association fénofibrate/substance vitaminique E présente une synergie, en ce qui concerne la protection des lipoprotéines telles que les LDL vis-à-vis de l'oxydation, quand les deux conditions suivantes sont remplies :

(i) le fénofibrate, qui intervient dans ladite association, a été préalablement co-micronisé avec un agent tensio-actif solide ; et,

(ii) le rapport Ra précité est compris entre 0,33 et 2 mg/IU.

Selon un premier aspect de l'invention, l'on préconise une nouvelle association de fénofibrate et d'une substance vitaminique E, qui est caractérisée en ce qu'elle comprend :

(a) un co-micronisat de fénofibrate avec un agent tensio-actif solide, et
(b) une substance vitaminique E choisie parmi l'ensemble constitué par les tocophérols, leurs esters avec des acides organiques et leurs mélanges,

avec les deux conditions suivantes :

(1) ledit co-micronisat contient 33 à 200 mg de fénofibrate et la quantité de ladite substance vitaminique E représente 100 à 600 IU, et
(2) le rapport (Ra) de la quantité de fénofibrate exprimée en mg à la quantité de ladite substance vitaminique E exprimée en IU est compris entre 0,33 et 2 mg/IU.

Selon un second aspect de l'invention, on préconise une nouvelle utilisation de ladite association fénofibrate co-micronisé/substance vitaminique E pour la préparation d'un médicament destiné à une utilisation en thérapeutique vis-à-vis des maladies athéromateuses et de leur prévention.

***Description détaillée de l'invention***

Par commodité dans la présente description, par l'expression "fénofibrate co-micronisé" on entend le fénofibrate préalablement co-micronisé avec un agent tensio-actif solide, et par "fénofibrate non co-micronisé" le fénofibrate qui a été micronisé en l'absence d'agent tensio-actif solide.
Par ailleurs, sur le plan thérapeutique, l'expression "association fénofibrate co-micronisé/substance vitaminique E" englobe selon l'invention soit une médication comprenant l'administration séparée (en générale simultanée) de fénofibrate co-micronisé et de substance vitaminique E, soit une médication comprenant l'administration d'une composition renfermant à la fois les deux ingrédients actifs.
Par "substance vitaminique E" on entend ici :

(i) une substance appartenant à l'ensemble des α-, β-, γ-, δ-, $\zeta_1$-, $\zeta_2$- et η-tocophérols, d'une part, et leurs éventuelles formes d1, d et 1, d'autre part ;
(ii) les esters correspondants obtenus avec des acides organiques ; et,
(iii) leurs mélanges.

De façon avantageuse, ladite substance vitaminique E sera choisie parmi les d1-α-tocophérol, d-α-tocophérol, acétate de d1-α-tocophérol et acétate de d-α-tocophérol, la substance vitaminique E préférée selon l'invention étant l'acétate de d1-α-tocophérol.
Quand on utilise l'acétate de d1-α-tocophérol ou quand on exprime la quantité de toute autre substance vitaminique E en poids équivalent d'acétate de d1-α-tocophérol, le rapport Ra (en mg/IU) précité, devient un rapport pondéral Rw eu égard à la relation (1) ci-dessus. Quand on tient compte de ce rapport pondéral Rw, il faut selon l'invention que, dans l'association fénofibrate co-micronisé/substance vitaminique E, le rapport

$$Rw = \frac{\text{quantité de fénofibrate (mg)}}{\text{quantité de substance vitaminique E exprimée en équivalent d'acétate de d1-}\alpha\text{-tocophérol (mg)}}$$

soit compris entre 0,33 et 2.
Le co-micronisat fénofibrate/agent tensio-actif solide contient en général 0,75 à 10,5 parties en poids d'agent tensio-actif solide pour 100 parties en poids de fénofibrate et a une granulométrie inférieure à 15 µm, de préférence inférieure ou égale à 10 µm et mieux inférieure ou égale à 5 µm.
L'agent tensio-actif solide que l'on préfère ici est le laurylsulfate de sodium (LSNa), comme indiqué dans le document EP-A-0 330 532 précité. De façon pratique, on utilisera ici pour la réalisation du co-micronisat 1 à 7 parties en poids de LSNa pour 100 parties en poids de fénofibrate.
L'association selon l'invention peut être administrée sous une forme quelconque. On préfère en particulier, quand le mode d'administration est la forme orale, faire appel à des gélules ou à des comprimés.
En général, pour l'administration de l'association fénofibrate co-micronisé/substance vitaminique E, et en particulier pour celle de l'association fénofibrate co-micronisé/acétate de d1-α-tocophérol préférée selon l'invention, on prévoit deux solutions dans lesquelles Ra est toujours compris entre 0,33 et 2 mg/IU :

(α) l'administration quotidienne séparée, sous forme de gélules, (a) de 33 à 200 mg de fénofibrate co-micronisé avec le laurylsulfate de sodium en association avec un excipient physiologiquement acceptable, et (b) de 100 à 600 IU de substance vitaminique E (de préférence 100 à 600 mg d'acétate de d1-α-tocophérol) en association avec un excipient physiologiquement acceptable ; et,

(β) l'administration quotidienne d'une forme unique comprenant, d'une part, le fénofibrate co-micronisé avec LSNa et, d'autre part, la substance vitaminique E, selon les doses du point (α) ci-dessus, dans laquelle les véhicules des deux ingrédients actifs sont accolés ou juxtaposés (ce cas se rencontre notamment quand la forme unique comprend soit un comprimé de fénofibrate co-micronisé et un comprimé de substance vitaminique E accolés par un côté ou une face, soit une gélule renfermant simultanément ledit fénofibrate co-micronisé et ladite substance vitaminique E).

***Posologie***

La posologie quotidienne que l'on recommande selon l'invention consiste à administrer par voie orale une association de :

(a) un co-micronisat de 33 à 200 mg de fénofibrate avec 0,33 à 14 mg de LSNa, et
(b) 100 à 600 mg (i.e. 100 à 600 IU) d'acétate de d1-α-tocophérol,

dans cette association le rapport pondéral Rw étant compris entre 0,33 et 2 (i.e. Ra = 0,33-2 mg/IU).

De préférence, on administrera une composition renfermant ces deux ingrédients en présence d'un excipient, selon la solution du point (β) ci-dessus.

L'association selon l'invention est utile dans le traitement et la prévention des pathologies impliquant une oxydation des lipoprotéines, notamment les maladies athéromateuses, le diabète, l'hypertension artérielle essentielle, d'une part, et dans la prévention de la resténose, d'autre part, en ce sens qu'elle diminue, au niveau plasmatique, la teneur en lipide grâce au fénofibrate et qu'elle protège les lipoprotéines de faible densité, les LDL, vis-à-vis de l'oxydation grâce à la synergie fénofibrate co-micronisé/substance vitaminique E.

D'autres avantages et caractéristiques de l'invention seront mieux compris à la lecture qui va suivre de la description d'exemples de réalisation, de résultats pharmacologiques et d'essais comparatifs. Bien entendu l'ensemble de ces éléments n'est nullement limitatif mais est donné à titre d'illustration.

***Exemple 1***

On prépare une association comprenant, pour une administration quotidienne chez l'homme adulte :

- une gélule d'un co-micronisat (granulométrie inférieure ou égale à 5 µm) constitué de 200 mg de fénofibrate et de 4 mg de LSNa en association avec un excipient physiologiquement acceptable, et
- une gélule contenant 200 mg d'acétate de d1-α-tocophérol en association avec un excipient physiologiquement acceptable.

Rw = 1

***Exemple 2***

On prépare une association comprenant, pour une administration quotidienne chez l'homme adulte :

- une gélule d'un co-micronisat (granulométrie inférieure ou égale à 5 µm) constitué de 200 mg de fénofibrate et de 6 mg de LSNa en association avec un excipient physiologiquement acceptable, et
- une gélule contenant 400 mg d'acétate de d1-α-tocophérol en association avec un excipient physiologiquement acceptable.

Rw = 0,5

***Exemple 3***

On prépare une association comprenant, pour une administration quotidienne chez l'homme adulte :

- une gélule d'un co-micronisat (granulométrie inférieure ou égale à 5 µm) constitué de 200 mg de fénofibrate et de

6 mg de LSNa en association avec un excipient physiologiquement acceptable, et
- une gélule contenant 300 mg d'acétate de d1-α-tocophérol en association avec un excipient physiologiquement acceptable.

Rw = 0,67

***Exemple 4***

On prépare une association comprenant, pour une administration quotidienne chez l'homme adulte, une forme unique constituée de deux comprimés accolés, l'un contenant le co-micronisat (granulométrie inférieure ou égale à 5 µm) de 200 mg de fénofibrate et de 8 mg de LSNa en association avec un excipient physiologiquement acceptable et l'autre contenant 290 mg d'acétate de d1-α-tocophérol en association avec un excipient physiologiquement acceptable.
Rw = 0,69

***Exemple 5***

On prépare des gélules contenant chacune un mélange d'un co-micronisat (granulométrie inférieure ou égale à 5 µm) constitué de 100 mg de fénofibrate et de 3mg de LSNa, d'une part, et de 150 mg d'acétate de d1-α-tocophérol, d'autre part, en association avec un excipient physiologiquement acceptable, la dose quotidienne administrée chez l'homme adulte correspondant à 2 gélules.
Rw = 0,67

***Exemple 6***

On prépare une association comprenant, pour une administration quotidienne chez l'homme adulte :

- une gélule d'un co-micronisat (granulométrie inférieure ou égale à 5 µm) constitué de 200 mg de fénofibrate et de 6 mg de LSNa en association avec un excipient physiologiquement acceptable, et
- une gélule contenant 100 mg d'acétate de d1-α-tocophérol en association avec un excipient physiologiquement acceptable.

Rw = 2

***Exemple 7***

On prépare une association comprenant, pour une administration quotidienne chez l'homme adulte :

- une gélule d'un co-micronisat (granulométrie inférieure ou égale à 5 µm) constitué de 200 mg de fénofibrate et de 6 mg de LSNa en association avec un excipient physiologiquement acceptable, et
- deux gélules contenant chacune 300 mg d'acétate de d1-α-tocophérol en association avec un excipient physiologiquement acceptable.

Rw = 0,33

***Essais pharmacologiques et comparatifs***

Des essais ont été réalisés chez le rat mâle WISTAR adulte pesant environ 230 g, à raison de 5 ou 6 animaux par lot et par produit ou association à tester. Chaque animal a reçu par voie orale (intubation gastrique) pendant 3, 8, 15 ou 27 jours soit de l'huile de soja (2 ml/kg/j), soit (i) le fénofibrate co-micronisé (3 parties en poids LSNa pour 100 parties en poids de fénofibrate, comme indiqué à l'exemple 5 ci-dessus) ou non co-micronisé, (ii) l'acétate de d1-α-tocophérol ou (iii) leurs mélanges, en solution ou suspension dans la même quantité d'huile de soja (2ml/kg/j).
Les animaux de chaque lot ont reçu l'un des traitements suivants :

(A) huile de soja (lot contrôle) ;
(B) 37 mg/kg/j de fénofibrate co-micronisé, dans de l'huile de soja ;
(C) 55 mg/kg/j d'acétate de d1-α-tocophérol, dans de l'huile de soja ; et,
(D) association de 37 mg/kg/j de fénofibrate co-micronisé selon (B) et de 55 mg/kg/j d'acétate de d1-α-tocophérol selon (C) [Rw = 0,67], dans de l'huile de soja.

Pendant la durée de chaque essai, les animaux avaient un libre accès à la nourriture et à l'eau de boisson. A l'issue de chaque essai (3, 8, 15, 27 jours), les rats ont reçu la dernière dose puis ont été privés de nourriture. 5 heures après, les animaux ont été anesthésiés au pentobarbital sodique puis on a recueilli le sang, à partir de l'aorte abdominale, sur EDTA (1 mg/ml). Les plasmas ont alors été séparés par centrifugation à vitesse réduite, puis congelés à une température de -20 à -70°C pour analyses ultérieures.

Dans une première série, on a évalué les niveaux plasmatiques en cholestérol total, en phospholipides et en triglycérides. Les résultats obtenus, consignés dans le tableau I ci-après, montrent que le fénofibrate administré seul, provoque une diminution significative du cholestérol total plasmatique (variation de -31 à -40 %), diminue très faiblement les niveaux plasmatique des phospholipides et ne modifie pas ceux des triglycérides. Le fénofibrate, quand il est associé à l'acétate de d1-α-tocophérol génère les mêmes changements, alors que l'acétate de d1-α-tocophérol administré seul est inactif.

Les résultats du tableau I, qui sont la moyenne (n=6) ± erreur standard par rapport à la moyenne, sont donc conformes aux effets connus du fénofibrate et de l'acétate de d1-α-tocophérol sur les niveaux plasmatiques du cholestérol total, des phospholipides et des triglycérides.

Dans une seconde série on a évalué la protection de l'ensemble des lipoprotéines LDL+VLDL vis-à-vis de l'oxydation, en suivant la cinétique de la formation de diènes conjugués.

Dans ce but la fraction contenant les LDL et VLDL a été séparée par ultracentrifugation au moyen d'un dispositif comprenant un rotor à angle fixe. Dans ce but, la densité du plasma a été augmentée jusqu'à 1,050 g/ml par addition de KBr solide ; le plasma (4 ml) a été ensuite recouvert avec 4 ml d'une solution contenant NaCl (9 mg/ml), EDTA (1 mg/ml), KBr (à la densité de 1,050 g/ml) et soumis à une ultracentrifugation (45000 tours par minute ; 15°C ; 22 h) au moyen d'un dispositif BECKMAN 50 Ti ; le surnageant a été recueilli et conservé à l'obscurité à 4°C sous une atmosphère d'azote. Chaque échantillon de surnageant résultant a été dialysé à 4°C et à l'obscurité contre 2x5 l de sérum physiologique tamponné au phosphate (PBS) contenant 10 μM d'EDTA puis pendant 4 h contre PBS seul (pour éliminer KBr et EDTA). Les lipoprotéines (mélange LDL + VLDL) ainsi isolées ont été incubées à 37°C, pendant 10 heures au plus, dans 1 ml de PBS contenant 5 μM de $CuSO_4$, et la résistance à l'oxydation a été suivie en enregistrant la variation de l'absorbance, par rapport à l'absorbance initiale sans traitement avec $CuSO_4$, à 234 nm, toutes les 10 minutes pendant l'incubation. Les résultats obtenus montrent que, à la différence de l'enseignement de J.C. FRUCHART et al. (i.e. incubation de 24h), 5h d'incubation suffisent ici.

Les résultats relatifs à la protection des lipoprotéines (LDL + VLDL) vis-à-vis de l'oxydation ont été consignés dans le tableau II ci-après, dans lequel la vitesse (ou taux) de propagation est la pente de la portion de segment linéaire de la phase de propagation, et le temps de latence (en anglais : "lag time" ou "lag phase time") est l'intersection de cette portion de segment linéaire avec l'axe du temps en abscisses.

La vitesse de propagation est exprimée ici en

$$\frac{\Delta \text{ absorbance}}{\text{minute}} \times 1000$$

et le temps de latence est exprimé en minutes.

Les résultats du tableau II sont la moyenne (n=6, sauf indication contraire) ± erreur standard par rapport à la moyenne. Il montre que l'association selon l'invention fénofibrate co-micronisé/substance vitaminique E présente une synergie.

TABLEAU I

| TRAITEMENT | DUREE (jours) | (1) | (2) | (3) |
|---|---|---|---|---|
| A) Huile de soja (témoin) | 3 | 0,81 ± 0,03 | 1,33 ± 0,03 | 0,65 ± 0,10 |
| B) Fénofibrate co-micronisé (37 mg/kg/j) | 3 | 0,49 ± 0,02 * | 0,97 ± 0,02 * | 0,44 ± 0,03 |
| C) Acétate de d1-α-tocophérol (55 mg/kg/j) | 3 | 0,74 ± 0,03 * | 1,28 ± 0,04 | 0,72 ± 0,10 |
| D) B + C | 3 | 0,52 ± 0,02 * | 1,02 ± 0,01 * | 0,51 ± 0,03 |
| A) Huile de soja (témoin) | 8 | 0,67 ± 0,02 | 1,25 ± 0,04 | 0,83 ± 0,09 |

Notes :
(1) Cholestérol total (g/l)
(2) Phospholipides (g/l)
(3) Triglycérides (g/l)
(*) statistiquement significatif (p ≤ 0,05)

TABLEAU I (suite)

| TRAITEMENT | DUREE (jours) | (1) | (2) | (3) |
|---|---|---|---|---|
| B) Fénofibrate co-micronisé (37 mg/kg/j) | 8 | 0,46 ± 0,02 * | 1,09 ± 0,02 * | 0,43 ± 0,05 |
| C) Acétate de d1-α-tocophérol (55 mg/kg/j) | 8 | 0,69 ± 0,02 | 1,18 ± 0,05 | 0,69 ± 0,10 |
| D) B + C | 8 | 0,43 ± 0,01 * | 1,06 ± 0,02 * | 0,57 ± 0,10 |
| A) Huile de soja (témoin) | 15 | 0,66 ± 0,04 | 1,26 ± 0,04 | 0,82 ± 0,12 |
| B) Fénofibrate co-micronisé (37 mg/kg/j) | 15 | 0,41 ± 0,02 * | 1,23 ± 0,02 | 0,69 ± 0,05 |
| C) Acétate de d1-α-tocophérol (55 mg/kg/j) | 15 | 0,65 ± 0,04 | 1,30 ± 0,06 | 0,86 ± 0,08 |
| D) B + C | 15 | 0,39 ± 0,02 * | 1,23 ± 0,04 | 0,71 ± 0,08 |
| A) Huile de soja (témoin) | 27 | 0,70 ± 0,04 | 1,46 ± 0,05 | 1,16 ± 0,05 |
| B) Fénofibrate co-micronisé (37 mg/kg/j) | 27 | 0,43 ± 0,02 * | 1,29 ± 0,04 | 1,24 ± 0,23 |
| C) Acétate de d1-α-tocophérol (55 mg/kg/j) | 27 | 0,65 ± 0,03 | 1,35 ± 0,05 | 0,94 ± 0,11 |
| D) B + C | 27 | 0,41 ± 0,05 * | 1,30 ± 0,07 | 0,99 ± 0,11 |

Notes :
(1) Cholestérol total (g/l)
(2) Phospholipides (g/l)
(3) Triglycérides (g/l)
(*) statistiquement significatif ($p \leq 0,05$)

TABLEAU II

| TRAITEMENT | DUREE (a) | TEMPS DE LATENCE (b) | VITESSE DE PROPAGATION (c) |
|---|---|---|---|
| A) Huile de soja (témoin) | 3 | 39,2 ± 3,5 | 13,0 ± 0,4 |
| B) Fénofibrate co-micronisé (37 mg/kg/j) | 3 | 54,2 ± 4,7 (n=5) | 8,3 ± 0,2 (n=5) * |
| C) Acétate de d1-α-tocophérol (55 mg/kg/j) | 3 | 51,8 ± 2,9 (n=5) | 12,2 ± 0,4 (n=5) |
| D) B + C | 3 | 73,7 ± 7,9 * | 8,0 ± 0,3 * |
| A) Huile de soja (témoin) | 8 | 46,9 ± 2,8 | 14,1 ± 0,3 |
| B) Fénofibrate co-micronisé (37 mg/kg/j) | 8 | 128,4 ± 9,0 * | 6,3 ± 0,3 * |
| C) Acétate de d1-α-tocophérol (55 mg/kg/j) | 8 | 57,6 ± 3,4 | 12,4 ± 0,7 * |
| D) B + C | 8 | 155,3 ± 8,3 * | 6,0 ± 0,4 * |
| A) Huile de soja (témoin) | 15 | 46,1 ± 3,5 | 14,6 ± 0,7 |
| B) Fénofibrate co-micronisé (37 mg/kg/j) | 15 | 148,3 ± 11,0 * | 7,6 ± 0,4 * |

Notes :
(a) en jours
(b) en minutes
(c) en (Δabsorbance/minute) x 1000
(*) statistiquement significatif ($p \leq 0,05$)

TABLEAU II (suite)

| TRAITEMENT | DUREE (a) | TEMPS DE LATENCE (b) | VITESSE DE PROPAGATION (c) |
|---|---|---|---|
| C) Acétate de d1-α-tocophérol (55 mg/kg/j) | 15 | 62,8 ± 4,5 * | 12,7 ± 0,9 * |
| D) B + C | 15 | 226,1 ± 25,6 * | 5,4 ± 0,3 * |
| A) Huile de soja (témoin) | 27 | 58,0 ± 2,4 | 14,8 ± 0,5 |
| B) Fénofibrate co-micronisé (37 mg/kg/j) | 27 | 139,8 ± 3,4 * | 8,4 ± 0,5 * |
| C) Acétate de d1-α-tocophérol (55 mg/kg/j) | 27 | 67,4 ± 9,1 * | 11,9 ± 0,4 * |
| D) B + C | 27 | 206,6 ± 16,6 * | 6,3 ± 0,2 * |

Notes :
(a) en jours
(b) en minutes
(c) en (Δabsorbance/minute) x 1000
(*) statistiquement significatif ($p \leq 0,05$)

**Revendications**

**1.** Association de fénofibrate et d'une substance vitaminique E, caractérisée en ce qu'elle comprend :

(a) un co-micronisat de fénofibrate avec un agent tensio-actif solide, et
(b) une substance vitaminique E choisie parmi l'ensemble constitué par les tocophérols, leurs esters avec des acides organiques et leurs mélanges,

avec les deux conditions suivantes :

(1) ledit co-micronisat contient 33 à 200 mg de fénofibrate et la quantité de ladite substance vitaminique E représente 100 à 600 IU, et
(2) le rapport (Ra) de la quantité de fénofibrate exprimée en mg à la quantité de ladite substance vitaminique E exprimée en IU est compris entre 0,33 et 2 mg/IU.

**2.** Association selon la revendication 1, caractérisée en ce que le co-micronisat fénofibrate/agent tensio-actif solide comprend 0,75 à 10,5 parties en poids d'agent tensio actif solide pour 100 parties en poids de fénofibrate.

**3.** Association selon la revendication 1, caractérisée en ce que le co-micronisat fénofibrate/agent tensio-actif solide comprend 1 à 7 parties en poids d'agent tensio actif solide pour 100 parties en poids de fénofibrate.

**4.** Utilisation d'une association de fénofibrate et d'une substance vitaminique E pour la préparation d'un médicament vis-à-vis des pathologies impliquant une oxydation des lipoprotéines, notamment les maladies athéromateuses, le diabète, l'hypertension artérielle essentielle et la resténose et de leur prévention, ladite utilisation étant caractérisée en ce que le fénofibrate de ladite association est sous la forme d'un co-micronisat avec un agent tensio-actif solide.

**5.** Utilisation selon la revendication 4, caractérisée en ce que ladite association comprend :

(a) un co-micronisat de fénofibrate avec un agent tensio-actif solide, et
(b) une substance vitaminique E choisie parmi l'ensemble constitué par les tocophérols, leurs esters avec des acides organiques et leurs mélanges,

avec les deux conditions suivantes :

(1) ledit co-micronisat contient 33 à 200 mg de fénofibrate et la quantité de ladite substance vitaminique E

représente 100 à 600 IU, et
(2) le rapport (Ra) de la quantité de fénofibrate exprimée en mg à la quantité de ladite substance vitaminique E exprimée en IU est compris entre 0,33 et 2 mg/IU.

**6.** Utilisation selon la revendication 5, caractérisée en ce que le co-micronisat fénofibrate/agent tensio-actif solide comprend 0,75 à 10,5 parties en poids d'agent tensio actif solide pour 100 parties en poids de fénofibrate.

**7.** Utilisation selon la revendication 5, caractérisée en ce que le co-micronisat fénofibrate/agent tensio-actif solide comprend 1 à 7 parties en poids d'agent tensio actif solide pour 100 parties en poids de fénofibrate.

**8.** Utilisation selon la revendication 5, caractérisée en ce que ladite substance vitaminique E est choisie parmi les d1-α-tocophérol, d-α-tocophérol, acétate de d1-α-tocophérol et acétate de d-α-tocophérol.

**9.** Utilisation selon la revendication 5, caractérisée en ce que ladite substance vitaminique E est l'acétate de d1-α-tocophérol.

**Patentansprüche**

**1.** Mischung aus Fenofibrat und einer Vitamin E-Substanz,
**dadurch gekennzeichnet, dass sie**

a) ein Co-Micronisat von Fenofibrat mit einem festen obenflächenaktiven Wirkstoff und
b) eine aus der Gruppe von Tocopherolen, ihren Estern mit organischen Säuren und deren Mischungen ausgewählte Vitamin E-Substanz enthält,

mit den folgenden zwei Bedingungen:

1) das Co-Microsinat enthält 33 bis 200 mg Fenofibrat und eine 100 bis 600 IE entsprechende Menge der Vitamin E-Substanz und
2) das Verhältnis (Ra) der in mg ausgedrückten Menge von Fenofibrat zur in IE ausgedrückten Menge der Vitamin E-Substanz liegt zwischen 0,33 und 2 mg/IE.

**2.** Mischung nach Anspruch 1,
**dadurch gekennzeichnet, dass**
das Co-Microsinat aus Fenofibrat/festem oberflächenaktiven Wirkstoff 0,75 bis 10,5 Gew.-Teile des festen obenflächenaktiven Wirkstoffs pro 100 Gew.-Teile des Fenofibrats enthält.

**3.** Mischung nach Anspruch 1,
**dadurch gekennzeichnet, dass**
das Co-Micronisat aus Fenofibrat/festem oberflächenaktiven Wirkstoff 1 bis 7 Gew.-Teile des festen oberflächenaktiven Wirkstoffs pro 100 Gew.-Teile des Fenofibrats enthält.

**4.** Verwendung einer Mischung aus Fenofibrat und einer Vitamin E-Substanz zur Herstellung eines Arzneimittels gegen Erkrankungen, die mit einer Oxydation von Lipoproteinen zusammenhängen, insbesondere Atheromatose, Diabetes, essentielle, arterielle Hypertonie und Stenoserezidive und ihre Prävention, wobei diese Verwendung
**dadurch gekennzeichnet ist, dass**
das Fenofibrat der Mischung in der Form eines Co-Micronisats mit einem festen oberflächenaktiven Wirkstoff vorliegt.

**5.** Verwendung nach Anspruch 4,
**dadurch gekennzeichnet, dass die Mischung**

a) ein Co-Micronisat von Fenofibrat mit einem festen oberflächenaktiven Wirkstoff und
b) eine aus einer Gruppe bestehend aus Tocopherolen, ihren Estern mit organischen Säuren und deren Mischungen ausgewählte Vitamin E-Substanz enthält,

mit den folgenden zwei Bedingungen:

1) das Co-Micronisat enthält 33 bis 200 mg Fenofibrat und eine 100 bis 600 IE entsprechende Menge der Vitamin E-Substanz und
2) das Verhältnis (Ra) der in mg ausgedrückten Menge des Fenofibrats zur in IE ausgedrückten Menge der Vitamin E-Substanz liegt zwischen 0,33 und 2 mg/IE.

**6.** Verwendung nach Anspruch 5,
**dadurch gekennzeichnet, dass**
das Co-Micronisat aus Fenofibrat/festerm oberflächenaktiven Wirkstoff 0,75 bis 10,5 Gew.-Teile des festen oberflächenaktiven Wirkstoffs pro 100 Gew.-Teile des Fenofibrats enthält.

**7.** Verwendung nach Anspruch 5,
**dadurch gekennzeichnet, dass**
das Co-Micronisat aus Fenofibrat/festem oberflächenaktiven Wirkstoff 1 bis 7 Gew.-Teile festen oberflächenaktiven Wirkstoffs pro 100 Gew.-Teile Fenofibrat enthält.

**8.** Verwendung nach Anspruch 5,
**dadurch gekennzeichnet, dass**
die Vitamin E-Substanz aus dl-$\alpha$-Tocopherol, d-$\alpha$-Tocopherol, dl-$\alpha$-Tocopherolacetat und d-$\alpha$-Tocopherolacetat ausgewählt ist.

**9.** Verwendung nach Anspruch 5,
**dadurch gekennzeichnet, dass**
die Vitamin E-Substanz das dl-$\alpha$-Tocopherolacetat ist.

## Claims

**1.** A combination of fenofibrate and a vitamin E substance which comprises:

(a) a micronized mixture of fenofibrate with a solid surfactant, and
(b) a vitamin E substance selected from the group consisting of tocopherols, their esters with organic acids, and mixtures thereof,

with the following two conditions:

(1) said micronized mixture contains 33 to 200 mg of fenofibrate and the amount of said vitamin E substance represents 100 to 600 IU, and
(2) the ratio (Ra) of the amount of fenofibrate, expressed in mg, to the amount of said vitamin E substance, expressed in IU, is between 0.33 and 2 mg/IU.

**2.** A combination according to claim 1 wherein the micronized mixture of fenofibrate and solid surfactant comprises 0.75 to 10.5 parts by weight of solid surfactant per 100 parts by weight of fenofibrate.

**3.** A combination according to claim 1 wherein the micronized mixture of fenofibrate and solid surfactant comprises 1 to 7 parts by weight of solid surfactant per 100 parts by weight of fenofibrate.

**4.** Use of a combination of fenofibrate and a vitamin E substance for preparing a drug for the treatment and prevention of pathological conditions which involve oxidation of the lipoproteins, especially atheromatous diseases, diabetes, essential arterial hypertension and restenosis, wherein the fenofibrate of said combination is in the form of a micronized mixture with a solid surfactant.

**5.** Use according to claim 4 wherein said combination comprises:

(a) a micronized mixture of fenofibrate with a solid surfactant, and
(b) a vitamin E substance selected from the group consisting of tocopherols, their esters with organic acids, and mixtures thereof,

with the following two conditions:

(1) said micronized mixture contains 33 to 200 mg of fenofibrate and the amount of said vitamin E substance represents 100 to 600 IU, and
(2) the ratio (Ra) of the amount of fenofibrate, expressed in mg, to the amount of said vitamin E substance, expressed in IU, is between 0.33 and 2 mg/IU.

**6.** Use according to claim 5 wherein the micronized mixture of fenofibrate and solid surfactant comprises 0.75 to 10.5 parts by weight of solid surfactant per 100 parts by weight of fenofibrate.

**7.** Use according to claim 5 wherein the micronized mixture of fenofibrate and solid surfactant comprises 1 to 7 parts by weight of solid surfactant per 100 parts by weight of fenofibrate.

**8.** Use according to claim 5 wherein said vitamin E substance is selected from dl-α-tocopherol, d-α-tocopherol, dl-α-tocopherol acetate and d-α-tocopherol acetate.

**9.** Use according to claim 5 wherein said vitamin E substance is dl-α-tocopherol acetate.